# EUROPEAN PATENT APPLICATION

(11) **EP 3 973 871 A1**
(43) Date of publication of application: **30.03.2022**
(21) Application number: 20809303.9
(22) Date of filing: 19.05.2020
(51) Int. Cl.: A61B 5/1468, A61B 5/145, A61B 5/1486, A61B 5/155

(54) **ELECTROCHEMICAL BIOSENSOR COMPRISING CARBON NANOTUBE FOR MEASURING BIOSIGNALS AND METHOD FOR MANUFACTURING SAME**

(30) Priority: 20.05.2019 KR 20190059001
(71) Applicant: Sogang University Research Business Development Foundation, Seoul 04107 (KR); I-SENS, INC., Seoul 06646 (KR)
(72) Inventor: SHIN, Woonsup, Seoul 04107 (KR); QUAN, Yuzhong, Seoul 04107 (KR); HA, Eunhyeon, Seoul 04107 (KR); KIM, Suk-Joon, Seoul 06646 (KR); KANG, Young Jea, Seoul 06646 (KR); JEONG, In Seok, Seoul 06646 (KR); YANG, Hyunhee, Seoul 06646 (KR); KIM, Minki, Gyeonggi-do 06646 (KR)
(74) Representative: Arnold & Siedsma
(86) International application number: PCT/KR2020/006558
(87) International publication number: WO 2020/235919

(57) **Abstract**

The present invention provides an electrochemical biosensor comprising a carbon nanotube for measuring biosignals. The electrochemical biosensor for continuous glucose monitoring comprises an electrode to which a sensing film including an oxidoreductase, an electron transfer mediator, and a crosslinker is fixed, together with a carbon nanotube, wherein the oxidoreductase oxidizes a target substance and the electron thus generated in the oxidation process is transferred through the electron transfer mediator and the carbon nanotube, whereby the electrochemical biosensor can be used as a sensor having an excellent performance for continuous glucose monitoring.

## Description

### [TECHNICAL FIELD]

### Cross-reference to related application(s)

The present application claims the benefit of priority based on Korean Patent Application No. 10-2019-00590001 filed on May 20, 2019, and the entire contents disclosed in the document of the corresponding Korean Patent Application are incorporated as a part of this specification.

The present invention relates to a biosignal measuring electrochemical biosensor in which a carbon nanotube is introduced and thereby, the absorption, stability and electron transfer rate are increased and the reaction time is reduced and the linearity of response is improved, a method for preparing thereof.

### [BACKGROUND ART]

Diabetes is a very serious disease that affects 1 in 19 people worldwide, and it shows a trend that is further increasing with aging and changing eating habits. This diabetes is classified into type 1 diabetes in which blood sugar cannot be controlled because the pancreas does not secret insulin, and type 2 diabetes in which blood sugar cannot be controlled due to cellular insulin resistance despite insulin secretion, and when insulin secretion is abnormal in case of severe type 2 diabetes, it is classified as type 1.5. As such, when the blood sugar is not controlled and therefore the concentration of blood glucose is maintained at a high level, the risk of various complications (e.g.: myocardial infarction, stroke, retinopathy, renal failure, etc.) is significantly increased, and thus, a technology that helps patients to measure and manage the blood sugar by themselves is very much needed.

As a technology that allows patients to measure blood sugar by themselves during daily life, there is an SMBG (self monitoring blood glucose) technology. This technology is a method in which patients cause a small amount of bleeding in capillaries of fingertips through a needle and measure the concentration of glucose in the blood obtained through the bleeding through a blood sugar measuring sensor. Although this technology can measure blood sugar simply and accurately, it is difficult to observe the change in blood sugar level of patients because only the blood sugar concentration at a specific time can be known. In addition, there is a disadvantage that each time it is measured, patients have to bleed directly from fingertips, which causes pain. In case of type 1 diabetes of diabetes, there are many congenital causes, so it is necessary to measure and manage blood sugar from a young age, and therefore the SMBG technology which causes pain e very time it is measured can be a great burden for young diabetic. Furthermore, problems such as bacterial infection due to blood collection may occur, and a prescribed period is required in the process of introducing a blood sample into a chemically treated sensor, so there is a problem in that an error occurs in the measurement of the blood sugar level.

As a method for measuring a glucose concentration, various methods such as electrochemical method and method by infrared spectroscopy have been reported through papers. [Yokowama, K., Sode, K., Tamiya, E., Karube, I. Anal. Chim. Acta 1989, 218, 137;Rabinovitch, B., March, W. F., Adams, R. L. Diabetes Care 1982, 5, 254; G. M., Moses, R. G., Gan, I. E. T., Blair, S. C. Diabetes Res. Clin. Pract. 1988, 4, 177; D_Auria, S., Dicesare, N., Gryczynski, Z., Gryczynski, I.; Rossi, M.; Lakowicz, J. R. Biochem. Biophys. Res. Commun. 2000, 274, 727]

Conventionally, measurement by electrochemical methods have been used the most, and the most commonly used electrochemical measurement method is a method using an enzyme capable of oxidizing glucose. This electrochemical method is used in the commonly used SMBG technology. Research and development for technology to measure sugar concentration by the SMBG method are also being conducted worldwide to increase the accuracy of measurement and reduce errors. Moreover, unlike SMBG, which can measure only blood sugar at a specific point in time, continuous glucose monitoring sensor technology that can continuously monitor blood sugar trends has also been actively researched and several products have been released.

Dexcom, Abbott and Medtronic are representative companies that developed and released continuous blood sugar measuring devices, and G5, Libre, and Guardian have been released and sold, respectively. All of these products are based on electrochemical principles. However, even with same electrochemical sensor, there is a big difference in performance and stability depending on the type of enzyme, electron transport mediator and electrode used in the sensor.

As a commercially available continuous blood sugar measuring device, a method for measuring the sugar concentration by oxidizing hydrogen peroxide and a method using an electron transfer mediator chemically bound to a polymer are known. As such, a blood sugar sensor using an electrochemical principle usually comprises enzyme capable of oxidizing glucose and may comprise hydrogen peroxide or various kinds of electron transfer mediators to transfer electrons of the enzyme. In order to implement this method in a continuous blood sugar measurement system, the enzyme and electron transfer mediator consisting of the sensor must be stable under storage conditions or measurement conditions, and the response time must be short by responding quickly to changes in blood sugar.

In particular, when an electron transfer mediator chemically bound to a polymer is used, the electron transfer rate of the electron transfer mediator is very slow and low sensitivity is shown in many cases.

Accordingly, as a biosignal measuring electrochemical sensor, such as an electrochemical sensor for continuous blood sugar measurement, comprising an electron transfer mediator having a transition metal complex and a polymer, there has been an increasing demand for a biosignal measuring electrochemical sensor which exhibits an excellently increased electron transfer rate and has high sensitivity.

### [DISCLOSURE]

### [TECHNICLA PROBLEM]

Under this background, a problem to be solved by the present invention is to provide an electrochemical sensor for continuous blood sugar measurement having high sensitivity while exhibiting an excellently increased electron transfer rate as described above and a method for preparing the same.

However, the technical problem to be achieved by the present examples is not limited to the technical problem as described above and other problems may exist.

### [TECHNICAL SOLUTION]

As one aspect for achieving the above technical problem, the present invention relates to a sensing membrane used in a biosignal measuring electrochemical sensor comprising a carbon nanotube and a biosignal measuring electrochemical sensor comprising the same. Preferably, the biosignal measuring electrochemical sensor is an electrochemical sensor for continuous blood sugar measurement. The sensing membrane of the biosignal measuring electrochemical sensor and sensor according to one example of the present invention may comprise an enzyme, an electron transfer mediator and a carbon nanotube, and may further comprise an electrode and polyanionic polymer. Furthermore, a coating for mechanically and chemically strengthening and stabilizing these components may be comprised.

### [ADVANTAGEOUS EFFECTS]

The biosignal measuring electrochemical sensor comprising a carbon nanotube according to the present invention has a high reaction rate and accuracy by remarkably increasing the adsorption force, stability and electron transfer rate, so that electrons are easily transferred. Accordingly, there is an advantage in that it is possible to provide a sensor capable of quickly and accurately measuring a change in a biosignal such as blood sugar compared to a conventional electrochemical biosignal sensor.

### [BRIEF DESCRIPTION OF THE DRAWINGS]

FIG. 1 is a graph of measuring the cyclic voltammetry of the electrode comprising a carbon nanotube (CNT) according to Preparative example 1 and the electrode without it.
FIG. 2 is a graph of confirming the response to the glucose at a concentration of 0.02 - 0.1 nM with the electrode comprising a carbon nanotube (CNT) according to Preparative example 1 and the electrode without it.
FIG. 3 is a graph of confirming the response to the glucose at a concentration of 1 -5 mM with the electrode comprising a carbon nanotube (CNT) according to Preparative example 1 and the electrode without it.
FIG. 4 is a graph of confirming the response time when the glucose concentration is changed from 0.1 mM to 1 mM with the electrode comprising a carbon nanotube (CNT) according to Preparative example 1 and the electrode without it.
FIG. 5 is a graph of confirming the baseline current measured at a glucose concentration of 0 mM with the electrode comprising a carbon nanotube (CNT) according to Preparative example 1 and the electrode without it.
FIG. 6 is a graph of confirming the electrode performance change for 14 hours by coating nafion on the electrode comprising a carbon nanotube according to Preparative example 1 and then driving by applying 0.35 V vs Ag/AgCl in 0.5 mM glucose/PBS solution.
FIG. 7 is a graph of confirming the response to the glucose at a concentration of 1 - 5 mM with the electrode comprising a carbon nanotube (CNT) according to Preparative example 2 and the electrode without it.
FIG. 8 is a graph of confirming the initial stabilization effect of the sensor with the electrode comprising a carbon nanotube (CNT) according to Preparative example 2 and the electrode without it.
FIG. 9 is a graph of confirming the response current change of the sensor with the electrode comprising a carbon nanotube (CNT) according to Preparative example 2 and the electrode without it.
FIG. 10 is a graph of confirming the cyclic voltammetry of the electrode for each scan rate of the sensor with the electrode comprising a carbon nanotube (CNT) according to Preparative example 2 and the electrode without it.
FIG. 11 a and b are graphs showing the peak plot of the cyclic voltammetry of the electrode for each scan rate (FIG. 11a) and each scan rate^{1/2} (FIG. 11b) of the sensor with the electrode comprising a carbon nanotube (CNT) according to Preparative example 2.
FIG. 12 is a graph showing the peak plot of the cyclic voltammetry of the electrode for each can rate (FIG. 11a) and each scan rate^{1/2} (FIG. 11b) of the sensor with an electrode without a caron nanotube.

### [BEST MODE]

Hereinafter, the present invention will be described in more detail.

Herein, the term, "biosignal measuring" means quantitatively analyzing a specific material in a biosample, for example, a material in a biosample or living body such as blood sugar, cholesterol, protein, hormone and the like in blood. Preferably, the biosignal measuring may be blood sugar measurement. Therefore, one example of the biosignal measuring electrochemical biosensor of the present invention may be an electrochemical biosensor for blood sugar measurement, and preferably, an electrochemical biosensor for continuous blood sugar measurement may be exemplified. The sensing membrane according to the present invention means a membrane which senses the biosignal in this biosignal measuring electrochemical biosensor.

The sensing membrane for the biosignal measuring electrochemical biosensor according to the present invention is characterized by comprising a carbon nanotube as above. The term, "carbon nanotube" is a carbon allotrope composed of carbon present in large quantities on Earth, and is a material in which one carbon is combined with another carbon atom in a hexagonal honeycomb pattern to form a tube, and means a material in a very small area in which the diameter of the tube is extremely small. The carbon nanotube is known as a perfect new material hardly having defects among present materials which have excellent mechanical properties, electrical selectivity, excellent field emission properties, high-efficiency hydrogen storage medium properties, and the like, and is prepared by advanced synthetic technology, and it may be prepared by electrical discharge, pyrolysis, laser deposition, plasma chemical vapor deposition, thermochemical vapor deposition, electrolysis, flame synthesis, and the like as a synthetic method. The carbon nanotube according to the present invention may have a single-walled, double-walled or multi-walled form, and in some cases, it may have a rope form, but preferably, it may be a single-walled carbon nanotube, a multi-walled carbon nanotube or a blend of a single-walled carbon nanotube and a multi-walled carbon nanotube.

The carbon nanotube may be comprised in an amount of 1 to 20 % by weight based on the total weight of the sensor.

As such, when a carbon nanotube is comprised, the absorption, stability and electron transfer rate are significantly increased. As one embodiment, the electrochemical biosensor for continuous blood sugar measurement of the present invention may exhibit an electron transfer rate increased for example, about 10 times, preferably, 1.5 to 20 times, compared to a biosensor without a carbon nanotube. As one specific example, as the result that the present inventors have confirmed the cyclic voltammetry, responsivity to glucose, response time, baseline current and electrode performance change for 14 days using an electrochemical biosensor for continuous blood sugar measurement as a biosignal measuring electrochemical biosensor with or without a carbon nanotube, they could confirmed that the electrochemical biosensor for continuous blood sugar measurement having a sensing membrane comprising a carbon nanotube and an electrode according to the present invention can reach the maximum current within seconds and show 2 times higher responsivity and also increase the current linearly without reduction of response due to saturation even in a high-concentration glucose region, and secure the electrode performance for a long time, compared to the sensor without a carbon nanotube.

In addition, the sensing membrane according to the present invention may further comprise an oxidoreductase, an electron transfer mediator and a crosslinking material with the carbon nanotube. As one specific aspect, the carbon nanotube, oxidoreductase, electron transfer mediator and crosslinking material may be comprised in the biosignal measuring electrochemical sensor according to the present invention with an electrode and a polyanionic polymer by forming a blend together.

The oxidoreductase comprised in the biosignal measuring sensing membrane and sensor according to the present invention is a generic term for enzymes which catalyze the redox reaction of a living body, and herein, it means an enzyme that is reduced by reacting with a target substance to be measured, for example, in case of the biosensor, a target substance (for example, glucose) to be measured. Specifically, herein, the oxidoreductase plays a role of oxidizing glucose, and it has a structure in which an electron transfer mediator delivers electrons obtained by oxidizing glucose like this. Then, a target substance is quantified by measuring signals such as the current change. In addition, the carbon nanotube provides the high conductivity and increase the rate and efficiency of transfer of electrons, thereby composing a sensor with enhanced performance.

The oxidoreductase to be used herein may be one or more kinds selected from the group consisting of dehydrogenase, oxidase, and esterase, and according to the redox or detection target substance, an enzyme using the target substance as a substrate may be selected and used among enzymes belonging to the above enzyme group.

More specifically, the oxidoreductase may be one or more kinds selected from the group consisting of glucose dehydrogenase (GDH), glutamate dehydrogenase, glucose oxidase, cholesterol oxidase, cholesterol esterase, lactate oxidase, ascorbic acid oxidase, alcohol oxidase, alcohol dehydrogenase, bilirubin oxidase, and the like.

On the other hand, the oxidoreductase may comprise a cofactor which plays a role of storing hydrogen taken from the oxidoreductase from a target substance to be measured (for example, a target substance), and for example, it may be one or more kinds selected from the group consisting of flavin adenine dinucleotide (FAD), nicotinamide adenine dinucleotide (NAD), pyrroloquinoline quinone (PQQ), and the like.

For example, when the blood glucose concentration is to be measured, as the oxidoreductase, glucose dehydrogenase (GDH) may be used, and the glucose dehydrogenase may be flavin adenine dinucleotide- glucose dehydrogenase (FAD-GDH) comprising FAD as a cofactor and/or nicotinamide adenine dinucleotide-glucose dehydrogenase comprising FAD-GDH as a cofactor.

In a specific example, the available oxidoreductase may be one or more kinds selected from the group consisting of FAD-GDH (for example, EC 1.1.99.10, etc.), NAD-GDH (for example, EC 1.1.1.47, etc.), PQQ-GDH (for example, EC1.1.5.2, etc.), glutamate dehydrogenase (for example, EC 1.4.1.2, etc.), glucose oxidase (for example, EC 1.1.3.4, etc.), cholesterol oxidase (for example, EC 1.1.3.6, etc.), cholesterol esterase (for example, EC 3.1.1.13, etc.), lactate esterase (for example, EC 1.1.3.2, etc.), ascorbic acid oxidase (for example, EC 1.10.3.3, etc.), alcohol oxidase (for example, EC 1.1.3.13, etc.), alcohol dehydrogenase (for example, EC 1.1.1.1, etc.), bilirubin oxidase (for example, EC 1.3.3.5, etc.), and the like.

More preferably, the oxidoreductase is glucose dehydrogenase which can maintain the activity of 70% or more in a 37°C buffer solution for 1 week.

In addition, the redox mediator plays a role of delivering electrons obtained by reduction (glucose oxidation) of the oxidoreductase, and it may comprise a transition metal complex in which one or more ligands are coordinated to the transition metal and a polymer backbone such as one or more kinds selected from the group consisting of poly(vinylpyridine) (PVP) or poly(vinylimidazole) (PVI), and poly allyl glycidyl ether (PAGE), and selectively, a linker structure connecting the polymer backbone and transition metal complex.

Preferably, the transition metal may be one kind transition metal selected from the group consisting of Os, Rh, Ru, Ir, Fe and Co, and more preferably, it is Os. In addition, ligands are generally monodentate, bidentate, tridentate or quadridentate, and any ligand capable of coordinating with a known transition metal may be used without limitation, but may preferably a nitrogen-containing heterocyclic compound such as a pyridine and/or imidazole derivative. Moreover, multidentate ligands may comprise a multiple pyridine and/or imidazole ring (for example, bipyridine, biimidazole, etc.).

In addition, the crosslinking material available herein may be a dialdehyde compound, di-epoxide compound or the like, but not limited thereto.

On the other hand, the sensing membrane according to the present invention may further comprise one or more kinds of additives selected from the group consisting of a surfactant, an aqueous polymer, a tertiary ammonium salt, fatty acid, a thickener, and the like, for a role of a dispersing agent for dissolving a reagent, an adhesive for preparing a reagent, a stabilizer for long-term storage, and the like.

The surfactant may play a role to aliquot the composition evenly on the electrode to be aliquoted with a uniform thickness when the composition is aliquoted. As the surfactant, one or more kinds selected from the group consisting of Triton X-100, sodium dodecyl sulfate, perfluorooctane sulfonate, sodium stearate, and the like may be used. Herein, to properly perform a role of aliquoting the blend forming the sensing membrane with a uniform thickness, the surfactant may be contained in an amount of 3 to 100 parts by weight, for example, 30 to 70 parts by weight, based on 100 parts by weight of the oxidoreductase. For example, when an oxidoreductase with the activity of 700 U/mg is used, the surfactant may be contained in an amount of 30 to 70 parts by weight based on the 100 parts by weight of the oxidoreductase, and when the activity of the oxidoreductase is higher than this, the content of the surfactant may be adjusted lower than this.

The aqueous polymer may have a weight-average molecular weight of about 2,500 to 3,000,000, for example, about 5,000 to 1,000,000, to effectively perform a role of helping stabilization and dispersing of a support and an enzyme.

The thickener plays a role of firmly attach a reagent to the electrode. As the thickener, one or more kinds selected from the group consisting of nitrosol, diethylaminoethyl-dextran hydrochloride (DEAE-Dextran hydrochloride), and the like may be used. Herein, to firmly attach a redox polymer to the electrode, the thickener may be contained in an amount of 10 to 90 parts by weight, for example, 30 to 90 parts by weight, based on 100 parts by weight of the oxidoreductase. For example, when an oxidoreductase with the activity of 700 U/mg is used, the thickener may be contained in an amount of 30 to 90 parts by weight based on 100 parts by weight of the oxidoreductase, and when the activity of the oxidoreductase is higher than this, the content of the thickener may be adjusted lower than this.

As other aspect, the present invention provides a biosignal measuring electrochemical biosensor comprising the sensing membrane comprising a carbon nanotube. Preferably, the biosignal measuring electrochemical biosensor is an electrochemical biosensor for continuous blood sugar measurement.

Specifically, the electrochemical biosensor for continuous blood sugar measurement according to the present invention may further comprise an electrode and a polyanionic polymer.

In addition, as the working electrode, carbon, metal, platinum, or a metal electrode in which the electrode is not ionized with respect to an applied potential may be used.

Furthermore, in case of the electrochemical biosensor with 2 electrodes, as the counter electrode, a gold, platinum, silver or silver/silver chloride electrode may be used, and in case of the electrochemical biosensor of 3 electrodes comprising even a reference electrode, as the reference electrode, a gold, platinum, silver or silver/silver chloride electrode may be used.

Herein, the polyanionic polymer is comprised to play a role of preventing interfering species having an anionic property, and for example, it means a multi-anionic polymer having a plurality of sulfonyl groups or carboxylate groups such as Nafion or PSS (polystyrene sulfonate) and polyacrylate.

Moreover, in addition to this electrode and polyanionic polymer, the present invention may further comprise for example, an insulator, a substrate, a diffusion layer, a protection layer, and the like. In case of the electrode, 2 kinds of electrodes such as a working electrode and a counter electrode, and 2 kinds of electrodes such as a working electrode, a counter electrode and a reference electrode may be comprised. In one embodiment, the biosensor according to the present invention, may be an electrochemical biosensor produced by applying a blend comprising the aforementioned carbon nanotube, electron transfer mediator, oxidoreductase and crosslinking material, on a substrate equipped with at least two, preferably, two or three electrodes and then drying. For example, in the electrochemical biosensor, an electrochemical biosensor, in which a working electrode and a counter electrode are equipped on the opposite sides of the substrate each other, and the sensing membrane comprised according to the present invention is laminated on the working electrode, and an insulator, a diffusion layer and a protection layer are laminated on both sides of the substrate equipped with the working electrode and counter electrode in order.

As a specific aspect, the substrate may be made of one or more kinds selected from the group consisting of PET (polyethylene terephthalate), PC (polycarbonate) and PI (polyimide).

As a diffusion layer, one or more kinds selected from the group consisting of Nafion, cellulose acetate and silicone rubber, polyurethane, and polyurethane-based copolymer may be used, and as a protection layer, one or more kinds selected from the group consisting of silicone rubber, polyurethane, and polyurethane-based copolymer may be used, but not limited thereto.

This electrochemical biosensor according to the present invention has a characteristic that the response time is reduced and the linearity of response is enhanced by excellently increasing the absorption, stability and electron transfer rate.

### [MODE FOR INVENTION]

Hereinafter, the present invention will be described in more detail by the following examples. However, the following examples are illustrating the present invention only, but the contents of the present invention are not limited by the following examples.

### [Example]

### Preparative example 1: Preparation of electrochemical sensor for continuous blood sugar measurement comprising carbon nanotube according to the present invention

In order to prepare the electrochemical sensor comprising a carbon nanotube according to the present invention, a sensor was prepared by the method as follows. At first, an electron transfer mediator (PVI-Os(bpy)₂Cl), an oxidoreductase (glucose dehydrogenase) and a crosslinking material (polyethylene glycol diglycidylether) were dissolved using an aqueous or organic solvent, respectively, and each solution was prepared using a stirring and ultrasonic dispersion method, and then each solution prepared was mixed to prepare a mixed solution. On the other hand, separately from the solution comprising the electron transfer mediator (PVI-Os(bpy)₂Cl), oxidoreductase (glucose dehydrogenase) and crosslinking material (polyethylene glycol diglycidylether), a carbon nanotube dispersion was prepared. The carbon nanotube dispersion was prepared by dispersing a carbon nanotube (CNT) in a solvent with Triton-X purchased from sigma-aldrich as a non-ionic surfactant using water as the solvent. For the dispersion of the carbon nanotube, an ultrasonic dispersion method was used. The carbon nanotube dispersion prepared by the method was additionally mixed with a mixed solution of the electron transfer mediator (PVI-Os(bpy)₂Cl), oxidoreductase (glucose dehydrogenase) and crosslinking material (polyethylene glycol diglycidylether) and it was stirred for dispersion. By this method, finally, a mixed solution comprising the electron transfer mediator (PVI-Os(bpy)₂Cl), oxidoreductase (glucose dehydrogenase), crosslinking material (polyethylene glycol diglycidylether) and carbon nanotube was prepared.

Furthermore, in order to produce an electrochemical sensor for continuous blood sugar, the solution prepared by the method described above was coated on a carbon paste-printed electrode using a drop coating method, and then it was cured by crosslinking reaction at a room temperature for 24 hours. After curing, the prepared sensor was washed using distilled water.

### Comparative example 1: Preparation of electrochemical sensor for continuous blood sugar measurement without carbon nanotube

In order to prepare an electrochemical sensor without a carbon nanotube, a sensor was prepared by the method as follows.

An electron transfer mediator, an oxidoreductase and a crosslinking material were dissolved using an aqueous or organic solvent, respectively, and each solution was prepared using a stirring and ultrasonic dispersion method, and each solution prepared was mixed, and finally, a mixed solution comprising the electron transfer mediator, oxidoreductase and crosslinking material was prepared. Moreover, to produce an electrochemical sensor for continuous blood sugar, the solution prepared by the method described above was coated on a carbon paste-printed electrode using a drop coating method, and then it was cured by crosslinking reaction at a room temperature for 24 hours. After curing, the prepared sensor was washed using distilled water.

### Example 1: Comparison of cyclic voltammetry of electrochemical sensor for continuous blood sugar measurement with or without carbon nanotube

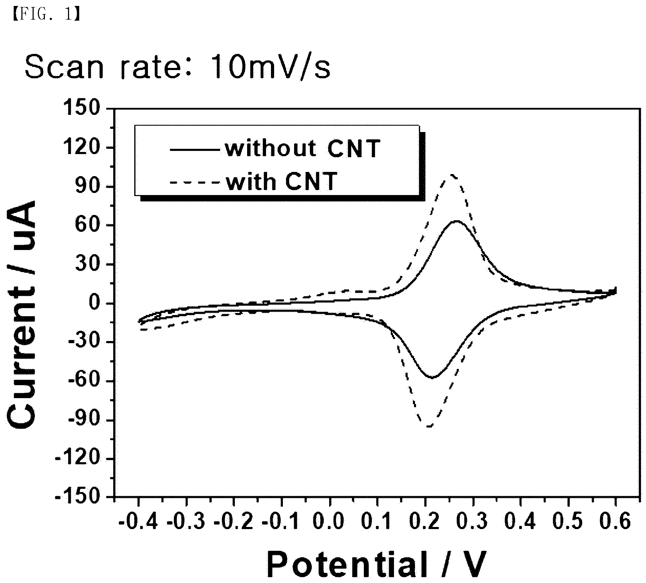

As a method for comparing the electron transfer performance of the electrode comprising a carbon nanotube with the electrode without a carbon nanotube, cyclic voltammetry was used. As a reference electrode for cyclic voltammetry, Ag/AgCl electrode was used. As a counter electrode, a platinum wire was used. As an electrolyte used when conducting cyclic voltammetry, physiological saline solution comprising phosphate buffer was used. When conducting cyclic voltammetry, as the scan rate converting the applied voltage, 10 mV/s was used. The order of applying voltage was first scan from high voltage to low voltage. This experimental result was shown in FIG. 1. As could be confirmed in FIG. 1, it could be found that the electrode comprising a carbon nanotube showed a higher redox peak than the electrode without it.

### Example 2: Comparison of responsivity to low concentration glucose of electrochemical sensor for continuous blood sugar measurement with or without carbon nanotube

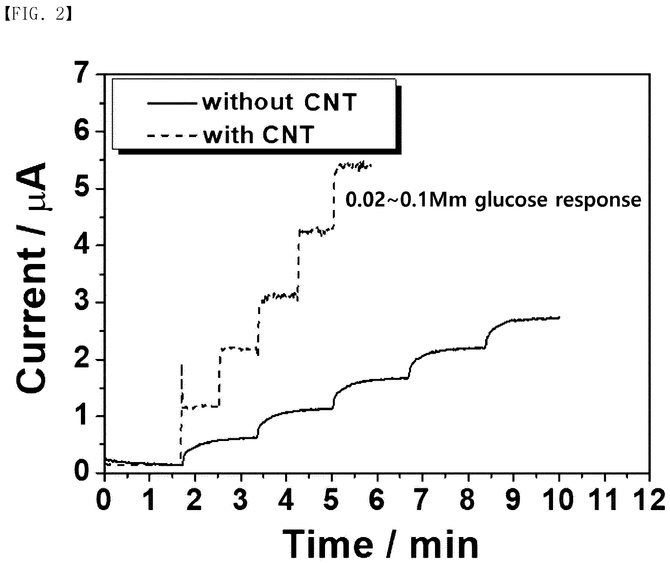

In order to compare the responsivity in a low glucose concentration range of the electrode with a carbon nanotube with the electrode without a carbon nanotube, using the electrode with a carbon nanotube and electrode without a carbon nanotube prepared in Preparative example 1, chronoamperometry was performed in a low concentration glucose solution to compare the responsivity. As a reference electrode for chronoamperometry, Ag/AgCl electrode was used. As a counter electrode, a platinum wire was used. As the voltage applied when performing chronoamperometry, a voltage larger than the oxidation voltage measured in a graph measured by cyclic voltammetry was applied in the positive (+) direction. As an electrolyte, physiological saline solution comprising phosphate buffer was used. The glucose concentration to see the responsivity in a low concentration glucose range was 0.02 mM, 0.04 mM, 0.06 mM, 0.08 mM and, 0.1 mM, and the experiment was proceeded for 12 minutes. The result was shown in FIG. 2. As could be confirmed in FIG. 2, it could be confirmed that the electrode comprising a carbon nanotube according to the present invention showed the responsivity about 2 times higher than the electrode without it.

### Example 3: Comparison of responsivity in high concentration glucose of electrochemical sensor for continuous blood sugar measurement with or without carbon nanotube

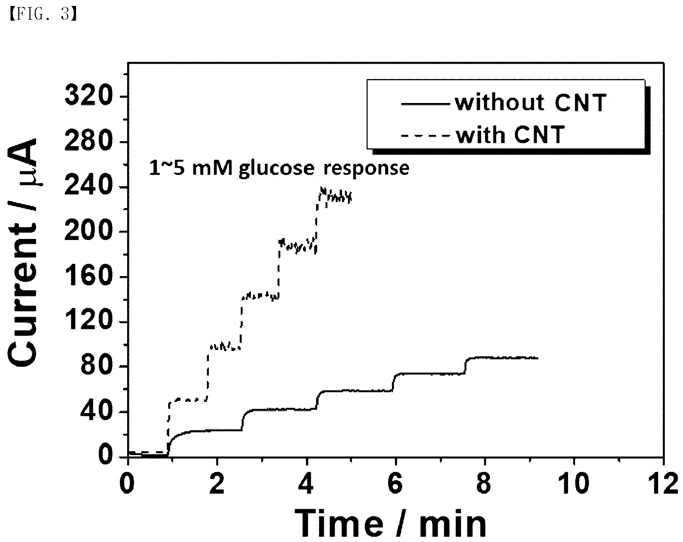

In order to compare the responsivity according to the presence or absence of the electrochemical sensor for continuous blood sugar measurement with or without a carbon nanotube in a high concentration glucose, proceeding by the same method as Example 2, it was tested using the concentration of glucose of 1 mM, 2 mM, 3 mM, 4 mM and 5 mM and the result was shown in FIG. 3. As could be confirmed in FIG. 3, it could be found that the electrode comprising a carbon nanotube showed the responsivity about 2.5 times higher than the electrode without it. In addition, it could be found that the electrode without a carbon nanotube was saturated in the high concentration region and thus the responsivity was reduced, but in the electrode comprising a carbon nanotube, the current was linearly increased in proportion to the glucose concentration.

### Example 4: Comparison of maximum current arrival time of electrochemical sensor for continuous blood sugar measurement with or without carbon nanotube

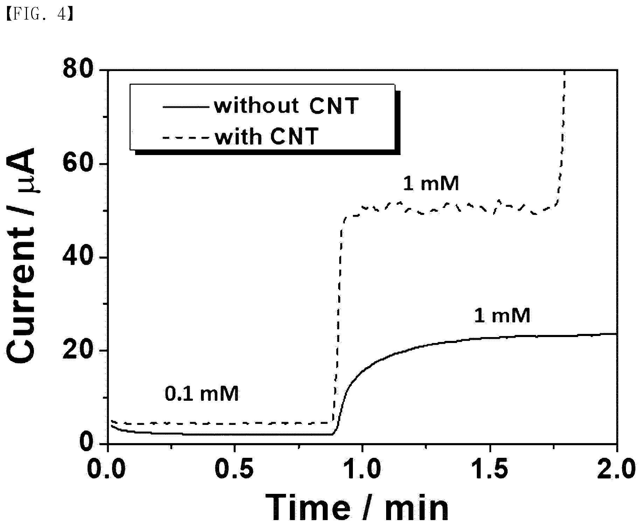

As a method for comparing the maximum current arrival time of the electrode comprising a carbon nanotube with the maximum current arrival time of the electrode without a carbon nanotube, chronoamperometry was used. Then, the reference electrode, counter electrode, applied voltage and electrolyte were performed in the same manner as Examples 2 and 3. When measuring the time of reaching the maximum current, the glucose solution at a concentration of 1M was added and finally, the glucose concentration was changed from 0.1 mM concentration to 1 mM, and the time for the current to reach the maximum when the concentration was changed was confirmed. Then, it was considered that the maximum current was reached when the noise occurred within the range of ± 10 % of the increased current, and the result was shown in FIG. 4. As could be found in FIG. 4, it could be found that in case of the electrode without a carbon nanotube, even when the glucose concentration was changed, the current was gradually increased and it took 30 seconds to 1 minute to stably reach the maximum current, but in case of the electrode comprising a carbon nanotube, the maximum current was reached within a few seconds.

### Example 5: Comparison of baseline current of electrochemical sensor for continuous blood sugar measurement with or without carbon nanotube

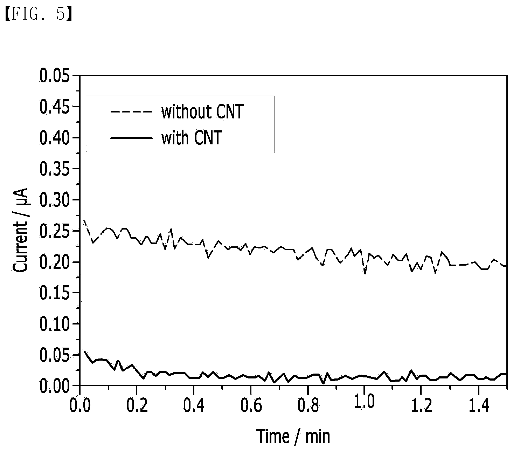

In order to confirm the baseline current measured in a concentration of 0 mM glucose with the electrode with or without a carbon nanotube, chronoamperometry was used. Then, the reference electrode, counter electrode, applied voltage and electrolyte were same as Examples 2 and 3. When measuring the baseline current, chronoamperometry was performed in a state where glucose was not added to the electrolyte. In addition, after applying a voltage and maintaining it for a sufficient time so that the current is maintained within a stable range, the magnitude of the maintained current is referred to as the baseline current. Then, the range was within ± 10 % of the current magnitude, and the result was shown in FIG. 5. As could be confirmed in FIG. 5, it could be confirmed that in case of the electrode without a carbon nanotube, a high baseline current of 0.1 *µ*A or higher flew even under the condition that there was no glucose at all, but it could be confirmed that the electrode comprising a carbon nanotube showed a low baseline current of 0.05 *µ*A or lower by completely oxidizing the electron transfer mediator in the electrode.

### Example 6: Confirmation of stability of responsivity by time of electrochemical sensor for continuous blood sugar measurement comprising carbon nanotube

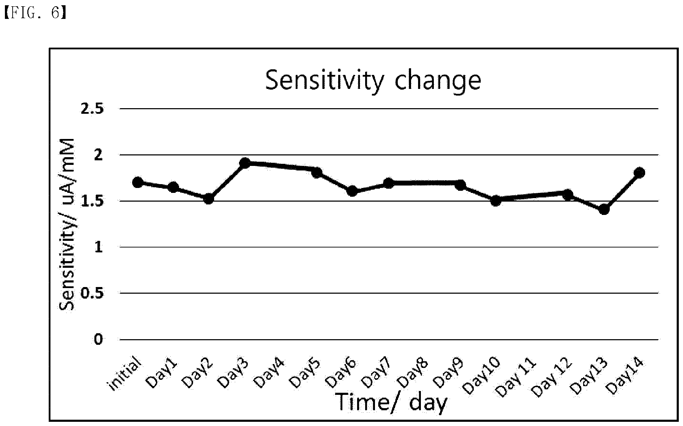

As a method for confirming the stability of the responsivity by time of the electrode comprising a carbon nanotube, chronoamperometry was used. Then, the reference electrode, counter electrode, applied voltage and electrolyte were same as Examples 2 and 3. The measurement was composed of storing a sensor under a specific condition for a certain time and measuring the responsivity of the sensor stored for a certain time by chronoamperometry, and the storing for a certain time and performing chronoamperometry were alternately configured. Thus, in the present test, nafion was coated on the electrode comprising a carbon nanotube and then 0.35 V vs Ag/AgCl was driven in 0.5 mM glucose/PBS solution to confirm the electrode performance change for 14 days. The responsivity was confirmed by a value of dividing the current measured by chronoamperometry in a glucose solution at a concentration of 1 mM or less by the glucose concentration. Under the condition of storing the sensor for a certain time, glucose at a concentration of 1M was added to the electrolyte so as to be finally a glucose solution at a concentration of 5 mM. The result was shown in FIG. 6. As shown in FIG. 6, it could be found that the electrode comprising a carbon nanotube according to the present invention showed an error in the sensitivity depending on the air temperature or measurement error, but in general, the stable sensitivity was maintained.

### Preparative example 2: Preparation of electrochemical sensor for continuous blood sugar measurement comprising carbon nanotube according to the present invention

In order to prepare the electrochemical sensor comprising a carbon nanotube according to the present invention, a sensor was prepared by the method as follows. At first, PVI-Os(bpy)₂Cl as an electron transfer mediator, an oxidoreductase (GDH) and PEGDGE as a crosslinking material were dissolved in distilled water, respectively, and each solution was prepared using a stirring and ultrasonic dispersion method, and then each solution prepared was mixed finally to prepare a mixed solution comprising the electron transfer mediator, oxidoreductase and crosslinking material.

On the other hand, separately from the solution comprising the electron transfer mediator, oxidoreductase and crosslinking material, a carbon nanotube dispersion was prepared. The carbon nanotube dispersion was prepared by dispersing a carbon nanotube (CNT) in a solvent with Triton-X purchased from sigma-aldrich as a non-ionic surfactant using water as the solvent. For the dispersion of the carbon nanotube, an ultrasonic dispersion method was used. The carbon nanotube dispersion prepared by the method was additionally mixed with a mixed solution of the electron transfer mediator, oxidoreductase and crosslinking material and it was stirred for dispersion. By this method, finally, a mixed solution comprising the electron transfer mediator, oxidoreductase, crosslinking material and carbon nanotube was prepared.

Furthermore, in order to produce an electrochemical sensor for continuous blood sugar, the solution prepared by the method described above was coated on a carbon paste-printed electrode using a drop coating method. As the electrode, a screen printed carbon electrode was used. Then, it was cured by crosslinking reaction in an oven maintained at 25 °C Celsius and 50% relative humidity at a room temperature for 24 hours. After curing, the prepared sensor was washed using distilled water.

### Example 7: Comparison of responsivity to high concentration glucose of electrochemical sensor for continuous blood sugar measurement with or without carbon nanotube-2

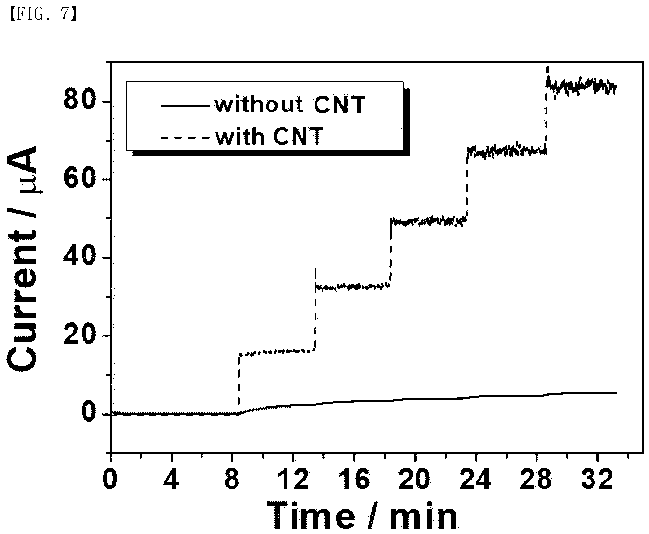

Using the electrode comprising a carbon nanotube prepared in Preparative example 2 and an electrode without it, the responsivity was compared by performing chronoamperometry in a glucose solution at a high concentration. As a reference electrode for chronoamperometry, Ag/AgCl electrode was used. As a counter electrode, a platinum wire was used. As the voltage applied when performing chronoamperometry, a voltage (0.4 V vs) larger than the oxidation voltage measured in a graph measured by cyclic voltammetry was applied in the positive (+) direction. As an electrolyte, physiological saline solution comprising phosphate buffer was used. The glucose concentration to see the responsivity in a low concentration glucose range was 1 mM, 2 mM, 3 mM, 4 mM and 5 mM, and the experiment was proceeded for 12 minutes. The result was shown in FIG. 7. As could be confirmed in FIG. 7, it could be confirmed that the electrode comprising a carbon nanotube according to the present invention showed the high responsivity, whereas the electrode without a carbon nanotube hardly showed the responsivity.

### Example 8: Comparison of initial stabilization effect with or without carbon nanotube

In order to compare the initial stabilization effect of the electrode comprising a carbon nanotube prepared in Preparative example 2 and an electrode without it, a test was conducted by the method as follows.

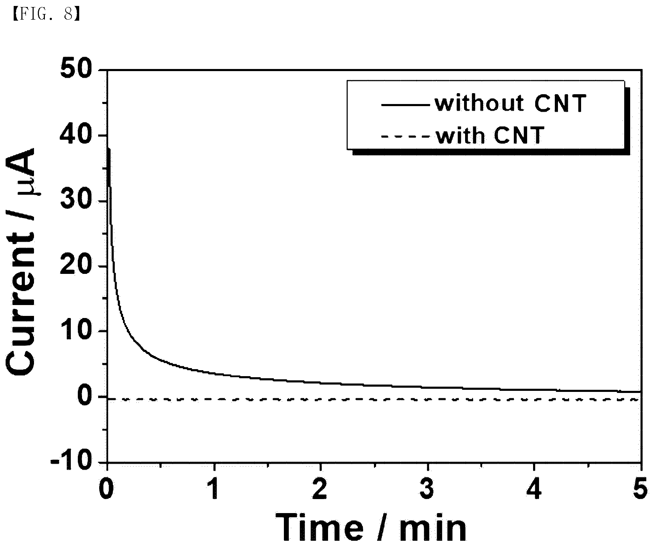

As a working electrode, an electrode comprising a carbon nanotube or an electrode without it was used, and as a reference electrode, Ag/AgCl electrode was used, and as a counter electrode, a platinum wire was used. As the voltage applied when performing chronoamperometry, a voltage (0.4 V vs) larger than the oxidation voltage measured in a graph measured by cyclic voltammetry was applied in the positive (+) direction. As an electrolyte, physiological saline solution comprising phosphate buffer was used. The glucose concentration of the physiological saline solution to see the stabilization tendency was 0 mM, and the experiment was proceeded for 5 minutes. The result was shown in FIG. 8. As could be confirmed in FIG. 8, it could be confirmed that the electrode comprising a carbon nanotube according to the present invention stabilized the current from the first voltage application, whereas the electrode without a carbon nanotube did not stabilize the current until about 3 minutes after the voltage application.

### Example 9: Comparison of response current change according to glucose concentration of electrochemical sensor for continuous blood sugar measurement with or without carbon nanotube

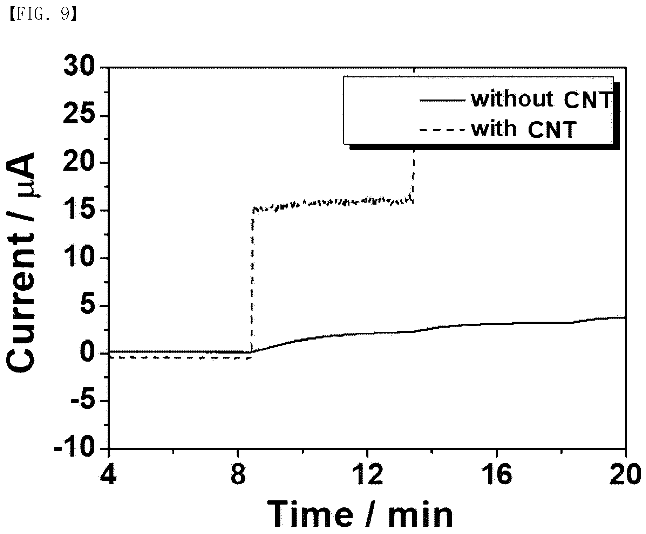

In order to compare the response current change of the electrode comprising a carbon nanotube prepared in Preparative example 2 and an electrode without it, a test was conducted by the method as follows. As a working electrode, an electrode comprising a carbon nanotube or an electrode without it was used, and as a reference electrode, Ag/AgCl electrode was used, and as a counter electrode, a platinum wire was used. As the voltage applied when performing chronoamperometry, a voltage (0.4 V vs Ag/AgCl) larger than the oxidation voltage measured in a graph measured by cyclic voltammetry was applied in the positive (+) direction. As an electrolyte, physiological saline solution comprising phosphate buffer was used. To compare the current change when the glucose concentration of physiological saline solution, the glucose concentration was changed from 0.1 mM to 1 mM, and the experiment was performed by applying 0.4 V vs Ag/AgCl for 2 minutes in total that are 1 minute at 0.1 mM and 1 minute at 1 mM. The result was shown in FIG. 9. As could be confirmed in FIG. 9, it could be confirmed that the electrode without a CNT took more than several minutes to reach the maximum current, but the electrode comprising a CNT had the fast rate of change in response of the electrode comprising a CNT as it reached the maximum current within a few seconds.

### Example 10: Comparison of cyclic voltammetry by scan rate of electrochemical sensor for continuous blood sugar measurement with or without a carbon nanotube

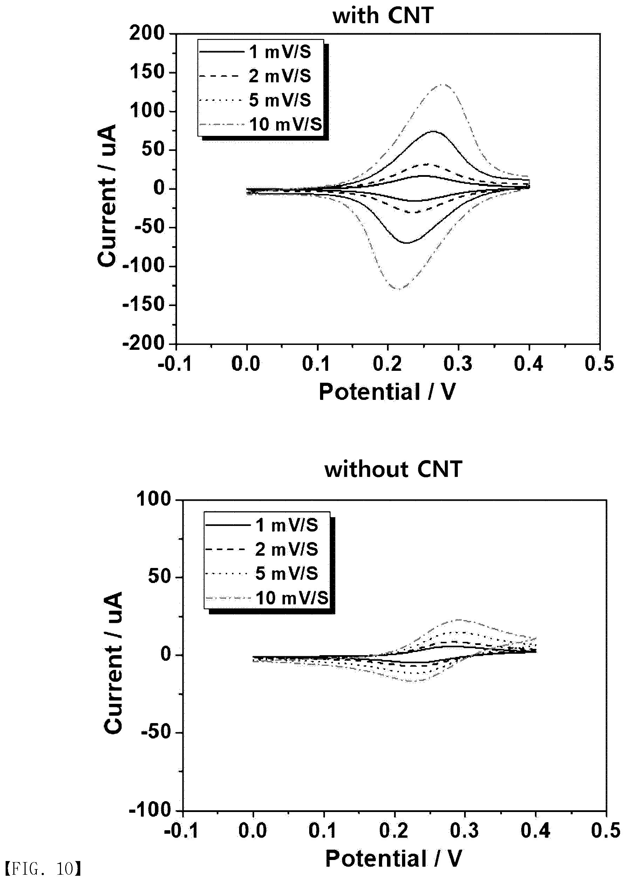

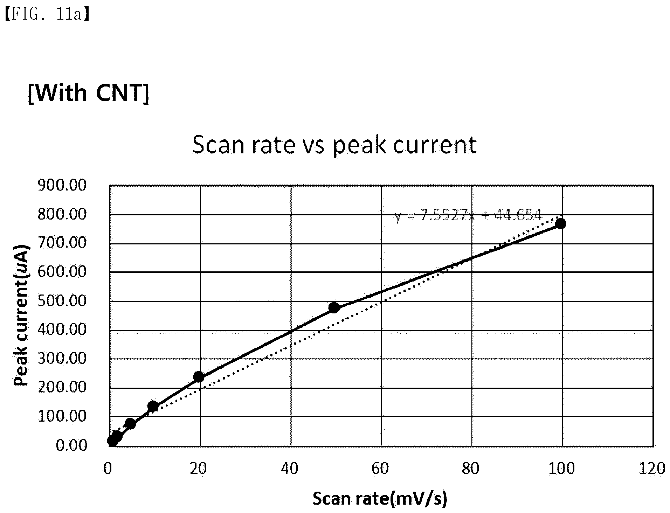

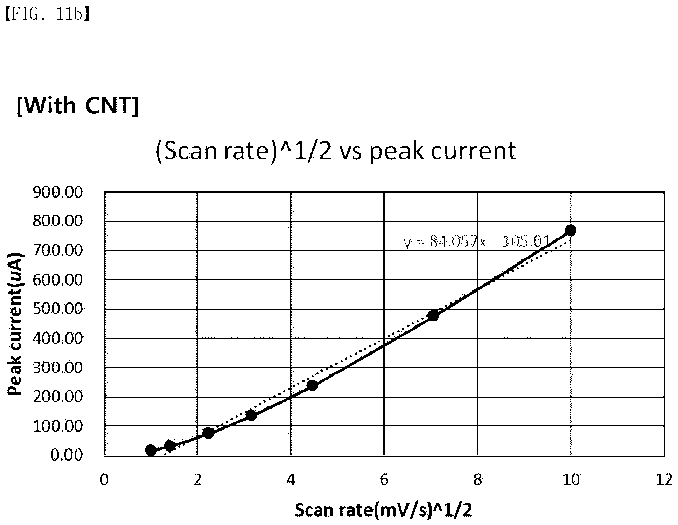

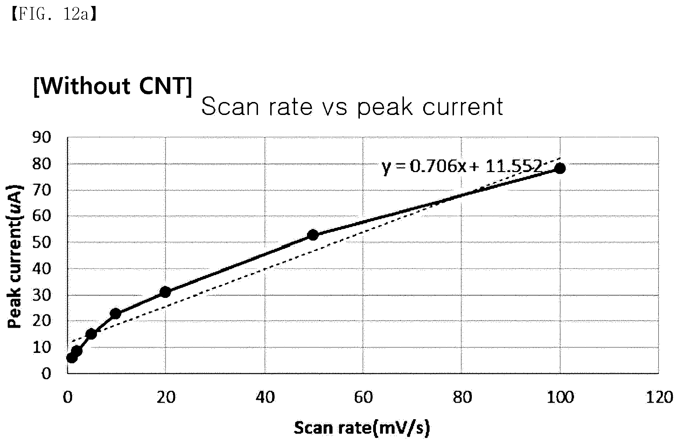

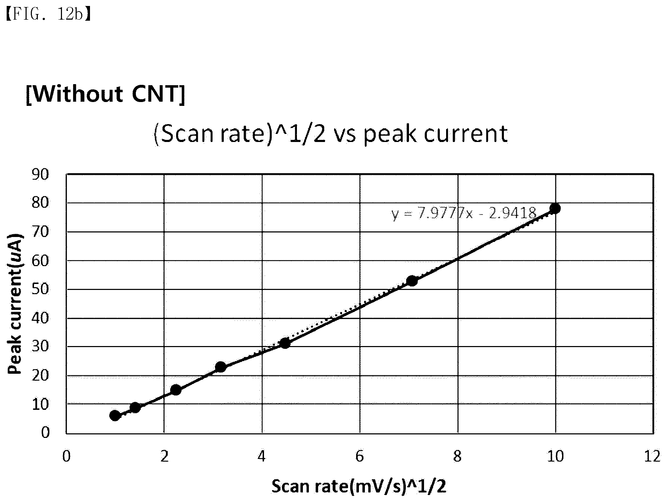

As a method for comparing the electron transfer performance of the electrode comprising a carbon nanotube with an electrode without a carbon nanotube, cyclic voltammetry was used. As a reference electrode for cyclic voltammetry, Ag/AgCl electrode was used. As a counter electrode, a platinum wire was used. As an electrolyte when performing cyclic voltammetry, physiological saline solution comprising phosphate buffer was used. When performing cyclic voltammetry, as the scan rate converting the applied voltage, 1, 2, 5 and 10 mV/s was used. The order of applying voltage was first scan from high voltage to low voltage. This experimental result was shown in FIGs. 10, 11 and 12. FIG. 10 is a graph showing the change in the current value according to the voltage change, and as could be confirmed in FIG. 10, it could be found that the electrode comprising a carbon nanotube showed a redox peak higher than the electrode without it at all scan rates. FIGs. 11a, b and 12a, b are graphs showing the experimental results of the case of comprising a carbon nanotube (FIGs. 11a and b) and the case of not comprising it (FIG. 12) by a peak plot, and it could be found that the electrode without a CNT followed the diffusion mechanism as the current magnitude of the CV peak is proportional to the 1/2 power of the scan rate, but in case of the electrode comprising a CNT, the current magnitude of the CV peak increased to a greater extent than the increase of the scan rate 1/2 power, and it was proportional to the 1 power of the scan rate at a scan rate of 10 mV/s or less, and therefore, the surface response was increased in the electrode comprising a CNT than the electrode without it.

## Claims

1. A sensing membrane for an electrochemical biosensor for biosignal measurement comprising a carbon nanotube.

2. The sensing membrane according to claim 1, wherein the electrochemical biosensor for biosignal measurement is an electrochemical biosensor for continuous blood sugar measurement.

3. The sensing membrane according to claim 1, wherein the carbon nanotube is comprised in an amount of 1 to 20 % by weight based on the total weight of the sensing membrane.

4. The sensing membrane according to claim 1, further comprising an electron transfer mediator, a crosslinking material and an oxidoreductase.

5. The sensing membrane according to claim 4, wherein the electron transfer mediator comprises a metal complex comprising one kind of transition metal selected from the group consisting of Os, Rh, Ru, Ir, Fe and Co and a monodentate or multidentate ligand, and a polymer backbone.

6. The sensing membrane according to claim 5, further comprising a linker structure which connects the polymer backbone and transition metal complex.

7. The sensing membrane according to claim 5, wherein the polymer backbone is one or more kinds selected from the group consisting of Poly(vinylpyridine) (PVP) and Poly(vinylimidazole) (PVI) and Poly allyl glycidyl ether (PAGE).

8. The sensing membrane according to claim 5, wherein the ligand is a heterocyclic compound of one or more kinds selected from the group consisting of pyridine and imidazole.

9. The sensing membrane for an electrochemical biosensor for continuous blood sugar measurement according to claim 4, wherein the oxidoreductase comprises one or more kinds of oxidoreductases selected from the group consisting of dehydrogenase, oxidase and esterase; or
one or more kinds of oxidoreductases selected from the group consisting of dehydrogenase, oxidase esterase and one or more kinds of cofactors selected from the group consisting of flavin adenine dinucleotide (FAD), nicotinamide adenine dinucleotide (NAD), and pyrroloquinoline quinone (PQQ).

10. The sensing membrane for an electrochemical biosensor for continuous blood sugar measurement according to claim 1, wherein the carbon nanotube is a single-walled carbon nanotube, a multi-walled carbon nanotube or a blend of a single-walled carbon nanotube and a multi-walled carbon nanotube.

11. An electrochemical biosensor comprising a sensing membrane for a biosignal measuring electrochemical biosensor according to any one of claim 1 to claim 10, an electrode, a crosslinking material and a polyanionic polymer.

12. The electrochemical biosensor according to claim 11, wherein the sensor is for continuous blood sugar measurement.

13. The electrochemical biosensor according to claim 11, further comprising a diffusion layer, a protection layer, an insulator and a substrate.

14. The electrochemical biosensor according to claim 11, wherein the electrode is 2 electrodes consisting of a working electrode and a counter electrode, or 3 electrodes consisting of a working electrode, a counter electrode and a reference electrode.
